(19) Europäisches Patentamt
European Patent Office
Office européen des brevets

(11) EP 0 844 237 B1

(12) **FASCICULE DE BREVET EUROPEEN**

(45) Date de publication et mention de la délivrance du brevet:
**31.05.2000 Bulletin 2000/22**

(51) Int Cl.[7]: **C07C 319/04**, C07C 323/52

(21) Numéro de dépôt: **97402474.7**

(22) Date de dépôt: **20.10.1997**

(54) **Procédé de synthèse d'esters de l'acide mercapto-3 propionique**

Verfahren zur Herstellung von 3-Mercaptopropionsäureestern

Process for the synthesis of esters of 3-mercaptopropionic acid

(84) Etats contractants désignés:
**AT BE CH DE DK ES FR GB IE IT LI NL SE**

(30) Priorité: **22.11.1996 FR 9614299**

(43) Date de publication de la demande:
**27.05.1998 Bulletin 1998/22**

(73) Titulaire: **ELF AQUITAINE EXPLORATION PRODUCTION FRANCE**
**92400 Courbevoie (FR)**

(72) Inventeur: **Arretz, Emmanuel**
**64000 Pau (FR)**

(74) Mandataire: **Leboulenger, Jean et al**
**Elf Atochem S.A.,**
**Département Propriété Industrielle,**
**Cedex 42 - La Défense 10**
**92091 Paris la Défense (FR)**

(56) Documents cités:
**DE-A- 3 614 065** **US-A- 3 346 516**

- **DATABASE WPI Section Ch, Week 8152 Derwent Publications Ltd., London, GB; Class A97, AN 81-95912D XP002035852 & JP 56 147 763 A (TOYO KASEI KOGYO CO LTD) , 16 novembre 1981**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 844 237 B1

## Description

La présente invention concerne la préparation d'esters de l'acide mercapto-3-propionique (MPA), par addition de l'hydrogène sulfuré à des esters de l'acide acrylique, selon la réaction (1) :

$$H_2S + CH_2 = CH\text{-}COOR \rightarrow HS\text{-}CH_2\text{-}CH_2\text{-}COOR \qquad (1)$$

L'ester de l'acide mercapto-3-propionique formé peut réagir sur l'ester de l'acide acrylique présent dans le milieu réactionnel pour donner l'ester de l'acide thio-3,3'-dipropionique selon la réaction (2) :

$$HS\text{-}CH_2\text{-}CH_2\text{-}COOR + CH_2 = CH\text{-}COOR \rightarrow S(CH_2\text{-}CH_2\text{-}COOR)_2 \qquad (2)$$

Le groupe R ci-dessus représente un groupe hydrocarboné alkyle droit ou ramifié pouvant contenir de 1 à 24 atomes de carbone et être notamment un groupe alkylaryle ou un groupe cyclohexyle. Le groupe R peut être par exemple méthyle, éthyle, propyle, butyle, pentyle, hexyle, heptyle, octyle, nonyle, décyle, undécyle, dodécyle.

On connait déjà dans la littérature brevets des procédés de synthèse d'esters de l'acide mercapto-3-propionique.

Ainsi J56147763 décrit un tel procédé par addition de $H_2S$ à des esters de l'acide acrylique, utilisant comme catalyseurs des résines échangeuses d'anions ayant comme groupes fontionnels des amines tertiaires ou des hydroxydes d'ammonium quaternaires.

Le brevet US 5008432 concerne l'addition de $H_2S$ à des composés insaturés dont la double liaison oléfinique est conjuguée avec un groupe attracteur d'électron, par exemple des esters de l'acide acrylique.

Cette addition est réalisée en présence d'un catalyseur basique choisi parmi l'oxyde de magnésium et les résines échangeuses d'anions basiques. Ces résines sont choisies parmi celles ayant, comme groupe fonctionnel, des amines tertiaires ou des ammoniums quaternaires.

Si une résine échangeuse d'anion est utilisée, la pression de la réaction est en général de 3103 à 6895 kPa.

L'exemple II décrit l'addition de $H_2S$ à l'acrylate de méthyle, sans solvant, à une pression réactionnelle de 3102,75 kPa et à une température de 73°C, en présence de résine Amberlyst A-21 (Rohm et Haas). Cette résine possède des groupes fonctionnels diméthylamino. La sélectivité indiquée est de 97,3% avec une conversion de 100%, le rapport molaire $H_2S$/acrylate de méthyle étant de 6,3/1.

L'exemple IV montre que les sélectivités obtenues sont supérieures aux sélectivités obtenues à plus basse pression (1931 kPa) selon J56147763.

Par ailleurs, le brevet EP 0168167 décrit la préparation d'une silice ou d'une alumine fonctionnalisée par la tétraméthylguanidine.

La silice ou l'alumine est mise en réaction avec de la 2-[3-(triéthoxysilyl)-propyl]-1,1,3,3,-tétraméthylguanidine afin de greffer ce radical par formation d'une liaison -O-Si ou -O-Al et élimination d'éthanol. Les supports solides ainsi fonctionnalisés ont été étudiés comme catalyseurs basiques dans la transestérification.

Le but de la présente invention est de trouver des conditions de mise en oeuvre de la réaction (1) telles que, tout en gardant un taux de conversion très élevé, la sélectivité en ester de l'acide mercapto-3-propionique soit meilleure que celle de l'art antérieur, notamment celle que l'on pourrait obtenir à partir de l'enseignement technique du document US 5008432.

Ce but est atteint en remplaçant les résines de l'art antérieur par un support solide fonctionnalisé par des groupes guanidine, à la condition que ceux-ci soient dépourvus d'hydrogène directement lié à un atome d'azote.

Ainsi la présente invention propose un procédé de synthèse d'ester de l'acide mercapto-3 propionique par réaction d'addition de $H_2S$ à l'ester correspondant de l'acide acrylique en présence d'un support solide possédant des groupes fonctionnels basiques, caractérisé en ce que les groupes fonctionnels sont des groupes guanidine, à la condition que ceux-ci soient dépourvus d'hydrogène directement lié à un atome d'azote.

Le support solide peut être tout support insoluble dans le milieu réactionnel comprenant l'ester acrylique, $H_2S$, l'ester mercaptopropionique formé et, éventuellement de faibles quantités du sulfure obtenu par la réaction (2).

Le support peut être par exemple de la silice,de l'alumine, ou une résine polymérique.

D'une manière générale, l'insolubilité des résines est obtenue par une réticulation du ou des polymères constituant le support polymérique.

Plus précisément, la présente invention propose un procédé de synthèse des esters de l'acide mercapto-3 propionique par réaction d'addition de $H_2S$ à l'ester correspondant de l'acide acrylique en présence d'un support solide possédant des groupes fonctionnels basiques, caractérisé en ce que ces groupes fonctionnels sont choisis parmi :

1° un radical guanidine de formule générale (C) :

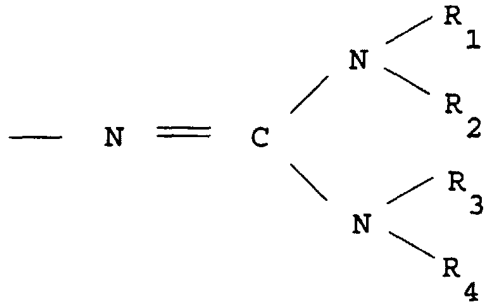

(C)

dans laquelle $R_1$, $R_2$, $R_3$, $R_4$ sont indépendamment l'un de l'autre des groupes hydrocarbonés tels que méthyle, éthyle, propyle, butyle, l'azote imine étant lié au support solide par une succession de liaisons chimiques,
2° un radical guanidine bicyclique de formule (D) :

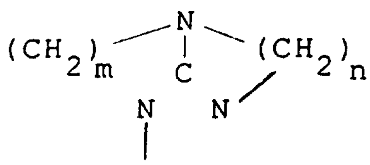

(D)

dans laquelle m et n valent chacun de 2 à 4, avec la condition que n soit inférieur ou égal à m, ce radical (D) étant lié au support solide par une liaison chimique ou une succession de liaisons chimiques partant de l'azote initialement N-H de la guanidine bicyclique correspondante.

Avantageusement, le radical (D) est choisi parmi les radicaux dérivés des guanidines bicycliques suivantes: 1,5,7-triazabicyclo [4.3.0] non-6-ène (m = 3, n = 2), 1,5,7-triazabicyclo 1,6,8-triazabicyclo [4.4.0] déc-5-ène (m = 3, n = 3), 1,4,6,-triazabicyclo[5.3.0] déc-7-ène (m = 4, n = 2), [3.3.0] oct-4-ène (m = 2, n = 2).

Ce procédé permet d'obtenir avec un excellent taux de conversion de l'ester acrylique, une meilleure sélectivité en ester de l'acide mercapto-3-propionique que les procédés de l'art antérieur, avec notamment une diminution concomitante de la teneur en sulfure diester dans le milieu réactionnel.

Ainsi d'une manière surprenante, tout se passe comme si les groupes fonctionnels guanidines augmentaient sélectivement la cinétique de la réaction (1) par rapport à la cinétique de la réaction (2).

L'augmentation de la sélectivité en ester de l'acide mercapto-3-propionique du procédé selon la présente invention se base ci-après sur la présentation d'exemples comparatifs comportant un étalonnage quantitatif de chromatogrammes (voir la partie expérimentale).

De préférence, la résine fonctionnalisée, à base de polystyrène divinylbenzène (PS-DVB), a la formule générale (I) suivante:

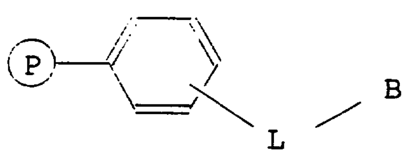

(I)

B étant un groupe choisi parmi les radicaux de formule générale (C), (D), L étant un radical organique linéaire ayant une longueur égale ou supérieure à celle du radical méthylène -($CH_2$-) et notamment le radical méthylène,

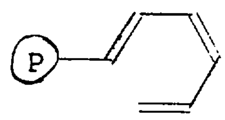

étant le support résine PS-DVB.

De préférence dans la formule générale (I):

- le radical (C) est substitué par L, ce dernier représentant alors un radical $-CH_2-$, et $R_1$, $R_2$, $R_3$ et $R_4$ représentant chacun un groupe méthyle,
- le radical (D) est substitué par L sur l'azote qui dans le composé bicyclique apparenté porte un hydrogène, avec la condition que L représente alors un radical $-(CH_2)_p-$, p entier valant 1 à 9,

Avantageusement, la résine fonctionnalisée a la formule générale (II):

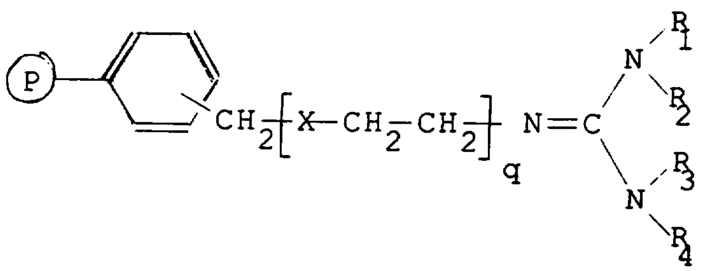

(II)

dans laquelle X représente un atome d'oxygène ou de soufre, q vaut 1 ou 2, $R_1$, $R_2$, $R_3$ et $R_4$ sont indépendamment l'un de l'autre choisis parmi les groupes méthyle, éthyle, propyle, butyle.

Avantageusement, dans la formule générale (II), $R_1$, $R_2$, $R_3$ et $R_4$ représentent chacun un groupe méthyle et q vaut 1.

De préférence, on met en oeuvre dans le procédé les résines à fonction guanidine bicyclique de formule générale (D) car elles montrent une plus grande stabilité chimique et thermique vis à vis du milieu réactionnel que les résines de formule générale (C).

De préférence, la résine fonctionnalisée a la formule générale (III):

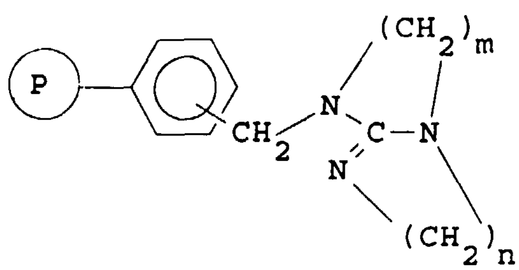

(III)

dans laquelle n vaut 2 ou 3 et m vaut 2, 3, ou 4, avec la condition que n soit inférieur ou égal à m.

De préférence, le rapport molaire $H_2S$/ester de l'acide acrylique doit être élevé pour favoriser encore plus la réaction (1) par rapport à la réaction (2) . Habituellement ce rapport molaire est de 3 à 10.

Pour augmenter ce rapport molaire dans le milieu liquide en contact avec la résine fonctionnalisée agissant comme catalyseur basique, on préfère soumettre le milieu réactionnel à une pression de $H_2S$ supérieure à la pression atmosphérique. De préférence, la pression réactionnelle est de 15 bars (1500 kPa) à 35 bars (3500 kPa).

Avantageusement, la réaction est effectuée à une température de 15°C à 80°C. De préférence la température du milieu réactionnel va de 15°C à 45°C.

Avantageusement, la quantité en poids de résine utilisée par rapport à la quantité en poids d'ester de l'acide acrylique mis en oeuvre est de 1 à 100 % et de préférence de 10 à 70 %.

La réaction peut être conduite dans une réacteur agité ou tubulaire, suivant un procédé discontinu, soit par

chargement des réactifs avant leur mise en réaction, soit par addition progressive de l'ester de l'acide acrylique après l'addition de l'hydrogène sulfuré, soit encore par addition simultanée des réactifs dans le réacteur, et enfin suivant un procédé continu avec addition contrôlée des réactifs

Les résines fonctionnalisées de formule générale (I) peuvent être obtenues ou préparées de la manière suivante :

1° Le groupe B est un radical de formule générale (C).

On connaît du brevet US 5340380 un procédé consistant à substituer le chlore d'une résine chlorométhylée de polystyrène-divinylbenzène par une guanidine substituée ou non et permettant d'obtenir des résines de formule générale (I.C) :

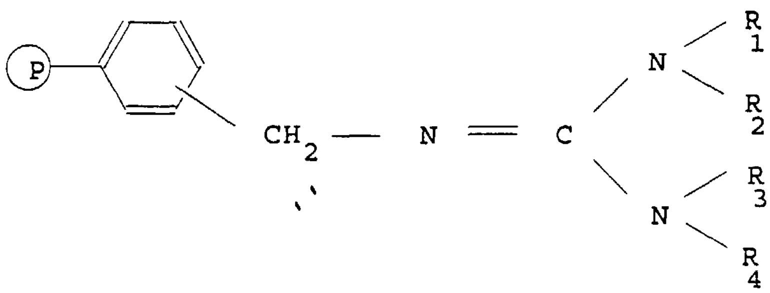

(I.C, L= $CH_2$)

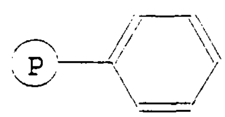

représentant le support solide, résine polystyrène-divinylbenzène de départ, $R_1$, $R_2$, $R_3$ et $R_4$ pouvant être chacun un hydrogène, un groupe alkyle ou un groupe aromatique.

Le brevet US 3346516 décrit aussi cette réaction d'une résine polystyrène-divinylbenzène chlorométhylée avec la guanidine ou la tétraméthyl guanidine en présence d'un alcool inférieur et un solvant de gonflement du copolymère PS-DVB comme le tétrahydrofuranne, le dioxane ou le diglyme.

Dans le brevet US 5028259, la tétraméthylguanidine est mise en contact avec une résine chlorométhylée de polystyrène-divinylbenzène dans un mélange de toluène et de tétrahydrofuranne.

Dans le brevet US 5340380 des guanidines sont mises en réaction avec ce même type de résines chlorométhylées en présence de soude dans un solvant constitué d'éthanol ou d'eau.

Cependant cette technique de fonctionnaliser une résine PS-DVB chlorométhylée par une guanidine est très limitée dans la pratique pour l'obtention de résines de formules (I.C) dont les radicaux guanidines portent des substituants $R_1$ à $R_4$ différents de quatre méthyles, dans la mesure ou seule la 1,1,3,3,-tétraméthylguanidine est actuellement commerciale.

De telles résines (I.C) dans lesquelles les groupes $R_1$ à $R_4$ sont tous différents de l'hydrogène peuvent être obtenues en utilisant des urées tétrasubstituées, souvent commercialisées, dans les conditions de préparation suivantes:

a) On commence par préparer une résine PS-DVB fonctionnalisée par des groupes amine primaire et ayant la formule générale (A)

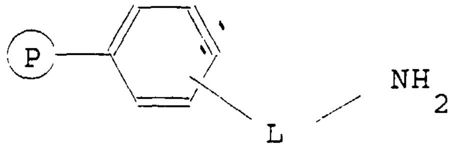

(A)

Celles-ci peuvent être obtenues par différentes techniques:

1/ On peut par exemple partir d'une résine de formule générale (J) :

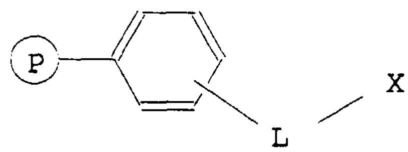

(J)

X étant un groupe partant notamment halogène ou tosylate obtenu à partir d'un groupe hydroxyle -OH, et L représentant notamment un radical $-(CH_2)_q-$, avec q entier valant de 1 à 9, y compris 2.

De préférence, lorsque L représente un seul méthylène, X est un atome de chlore. Dans ce cas, une méthode, décrite par D.H. Rich et S.K. Gurwara, J. Am. Chem. Soc., 1975, 97 - 1575-1579, consiste à faire réagir une résine PS-DVB chlorométhylée avec un excès d'ammoniac. Une autre voie est basée sur l'obtention de résine PS-DVB phtalimidométhylée qui est transformée par hydrazinolyse en résine à fonction amine primaire. Les deux méthodes d'accès à de telles résines phtalimido-méthylées sont décrites dans la publication de A.R. Mitchell, S.B.H. Kent, B.W. Erickson et R.B. Merrifield, Tetrahedron Letters N° 42, 1976, 3795-3798. L'une consiste à partir d'une résine PS-DVB qui par réaction avec le N-(chlorométhyl) phtalimide est directement convertie en résine phtalimidométhylée. L'autre méthode part d'une résine PS-DVB chlorométhylée qui est traitée par le phtalimide de potassium pour donner la résine correspondante phtalimidométhylée.

Quelques résines PS-DVB à fonction amine primaire de formule (A) dans laquelle L représente un méthylène sont commerciales.

Ainsi la Société PUROLITE propose deux résines macroporeuses, l'A-107 et l'A-109, tandis que la Société FLUKA a, dans son catalogue 1995-1996, deux résines gels : la résine 08564 PS réticulée avec 2 % de DVB et contenant 1,1 mmole de groupe $-NH_2$ par gramme de résine, et la résine 08566 PS réticulée avec 1 % de DVB et contenant 0,6 mmole de $-NH_2$ par gramme de résine.

La méthode au phtalimide de potassium est également applicable aux résines de formule (J) dans le cas où L est un radical organique linéaire d'une longueur supérieure à celle du radical méthylène, notamment $-(CH_2)_r-$, avec r valant un nombre entier supérieur à 1.

2/ On peut également partir d'une résine PS-DVB de formule (J) dans laquelle L représente un méthylène et X a la signification ci-dessus et de préférence représente un atome de chlore. La Demanderesse a trouvé que cette résine chlorométhylée peut être mise en réaction avec une alkanolamine ou une mercaptoalkylamine, sous forme d'alcoolate ou de thiolate alcalin, dans les conditions de la réaction de Williamson.

Si on met en oeuvre l'éthanolamine, on obtient des résines PS-DVB à fonction amine primaire avec des groupes fonctionnels $-CH_2-O-CH_2-CH_2-NH_2$ fixés aux supports résines PS-DVB.

De manière analogue, en partant du 2-amino éthanethiol hydrochlorure, on obtient les groupes fonctionnels $-CH_2-S-CH_2-CH_2-NH_2$.

Si on utilise le 2-(2-aminoéthoxy)éthanol, on obtient des résines PS-DVB à fonction amine primaire avec des groupes fonctionnels $-CH_2(-O-CH_2-CH_2)_2-NH_2$.

Enfin, en utilisant le 2-[(2-aminoéthyl)thio]éthanethiol, on obtient des groupes fonctionnels $-CH_2-(S-CH_2-CH_2)_2-NH_2$.

Cette mercaptoalkylamine de départ peut être préparée selon Iwakura et al., J. Polym. Sci. Part A, 2, 1964, 881-883 ou selon I Voronkov, M.G. et al., Chem. Heterocycl. Compd. (Engl. Transl.)15, 1979, 1183-1185.

Les conditions générales de la réaction de Williamson sont les suivantes :

L'alcanolamine ou la mercaptoalkylamine diluée dans du tétrahydrofuranne (THF) anhydre ou de la N-méthylpyrrolidone anhydre, est mise à réagir avec de l'hydrure de sodium en suspension dans le même solvant anhydre. Après formation de l'alcoolate de sodium ou du thiolate de sodium, la résine chlorométhylée est introduite dans le milieu réactionnel liquide.

b) Après obtention de la résine possédant des groupes amine primaire de formule générale (A), on fait réagir ces groupes amine primaire avec du chlorure de chloroformamidinium (sel de Vilsmeier) de formule générale (H) :

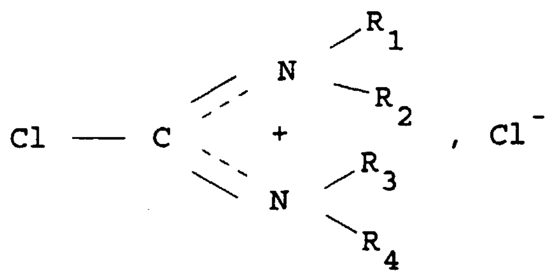

(H)

dans laquelle $R_1$, $R_2$, $R_3$ et $R_4$ sont indépendamment l'un de l'autre choisis parmi les groupes méthyle, éthyle, propyle, butyle,
pour obtenir une résine PS-DVB fonctionnalisée par un groupe guanidine et de formule générale (I.C) :

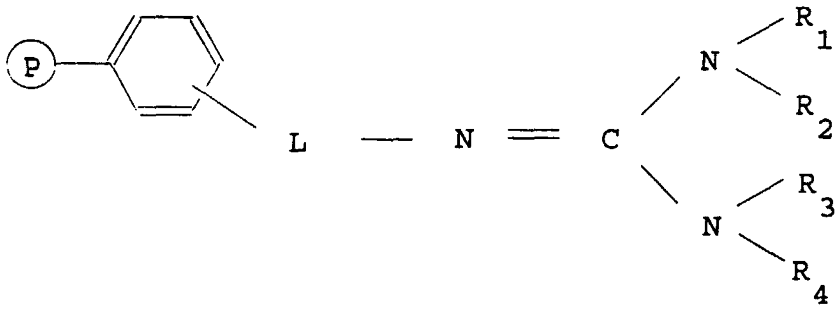

(I.C)

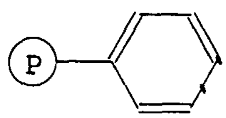

,

L, $R_1$ à $R_4$ ayant les mêmes significations que ci-dessus.

Les chlorures de chloroformamidinium (H) sont généralement obtenus à partir d'urées tétrasubstituées par réaction avec des composés électrophiles tels que phosgène, chlorure de thionyle, chlorure d'oxalyle, oxychlorure de phosphore, suivant des méthodes décrites dans la littérature, notamment:

| | |
|---|---|
| $COCl_2$ | H. Eilingsfeld, M. Seefelder, Angew.Chem., 72, 1960, 836. |
| $SOCl_2$ | H. Ulrich, A.A.R. Sayigh, Angew. Chem. Intern. Ed. Engl., 5, 1966, 704. |
| $(COCl)_2$ | T. Fujisawa et al., Chem. Lett.,1982, 1891. |
| $POCl_3$ | H. Bredereck, K. Bredereck, Chem. Ber., 94, 1961, 2278. |

Généralement, on part de quantités stoechiométriques d'urées tétrasubstituées et de composés chlorés électrophiles et on opère en présence d'un solvant tel que le tétrachlorure de carbone dans le cas du chlorure d'oxalyle, soit sans solvant avec le phosgène ou le chlorure de thionyle, à une température généralement de 0°C à 40°C, et en atmosphère anhydre pour éviter toute hydrolyse.

Avantageusement, on choisit les urées tétrasubstituées parmi la tétraméthylurée, la tétraéthylurée, la tétra n-propylurée et la tétra n-butylurée.

Les chlorures de chloroformamidinium (H) sont mis généralement dans un solvant tel que le toluène ou l'acétonitrile. Leurs réactions avec les résines à fonction amine primaire (A) sont effectuées en présence d'une base, de préférence en présence d'un excès de base.

Si la base est la triéthylamine (TEA), on opère généralement avec un excès molaire de TEA de 10 à 50% par rapport aux chlorures de chloroformamidinium (H). Ces derniers sont généralement en excès molaire de 10 à 100% par rapport au nombre de moles de fonction amine primaire, pour transformer la totalité de ces dernières en fonction

guanidine.

2° Dans la formule générale (I) le groupe B est un radical de formule (D)

(a) On commence par préparer une résine de formule générale (J) comme au point 1° a) ci-dessus, L représentant un radical $-(CH_2)_p-$, p entier valant 1 à 9 et X étant un chlore ou un brome.

(b) La résine halogénée ci-dessus est mise en réaction avec une guanidine bicyclique choisie notamment parmi le 1,5,7-triazabicyclo [4.3.0]non-6-ène (m = 3, n = 2) , le 1,5,7-triazabicyclo [4.4.0]déc-5-ène (TBD) (m (m = 3, n = 3), le 1,6,8-triazabicyclo [5.3.0] déc-7-éne (m = 4, n = 2), le 1,4,6-triazabicyclo [3.3.0] oct-4-éne (m = 2, n = 2).

La préparation de ces guanidines bicycliques est décrite dans les brevets GB 826837 et EP 0198680. La réaction est conduite d'une manière analogue au procédé de M. Tomoi et al, J.M.S. Pure Appl. Chem. A29(3), 1992, 249-261, en particulier page 251 (" Preparation of Polystyrene-Supported TBD).
On obtient ainsi une résine PS-DVB fonctionnalisée par un groupe guanidine bicyclique de formule générale (I.D.) :

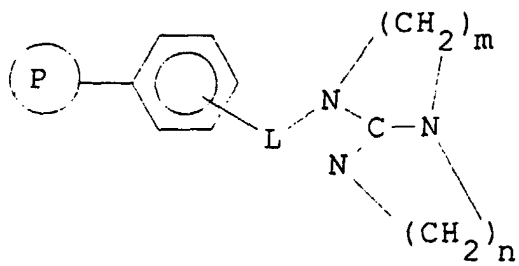

(I.D)

L représentant un radical $-(CH_2)_p-$ avec p entier valant 1 à 9.
Le procédé de Tomoi et al, J. Macromol. Sci. Pure Appl. Chem. A29(3) 1992, 249-261, consiste à faire réagir le sel de lithium du TBD sur une résine chlorométhylée.
Un mode opératoire simplifié a été étudié dans le cadre de la présente invention pour préparer des quantités plus importante de résines à fonction -TBD en faisant réagir directement le 1,5,7-triaza-bicyclo [4.4.0] déc-5-ène en excès sur de la résine PS-DVB chlorométhylée dans du THF anhydre comme solvant.
L'efficacité catalytique des résines utilisées dans la présente invention se trouve améliorée lorsqu'on les utilise sèches.
La présente invention sera mieux comprise grâce à la partie expérimentale ci-après, qui comprend notamment une description de l'appareillage utilisé, ce dernier étant schématiquement représenté sur la figure unique annexée.

**PARTIE EXPERIMENTALE**

**I. Préparation de résines polystyrène-divinylbenzène à fonction guanidine.**

La résine de base PS-DVB chlorométhylée qui est utilisée est de type macroporeux. Elle a les caractéristiques suivantes :

Surface spécifique : 22,5 $m^2$/g de résine
Diamètre moyen des pores : 20 Å
Volume des pores : 69%
chlorométhylée avec un taux de chlore de 19,32% en poids par rapport au poids total.

Cette résine contient donc 5,44 méq de Cl/g de résine.

**I.1 Obtention de résines PS-DVB de formule (I.D.) à fonction 1,5,7-triazabicyclo[4.4.0] déc-5-ène (TBD) avec L = -$CH_2$-.**

Mode opératoire:

On pèse 20 g de résine chlorométhylée sèche (5,44 méq de Cl/g de résine). La charge de résine contient 0,109 mole de Cl. On la met en contact sous atmosphère d'azote avec 30 g (0,216 mole) de TBD dilués dans 285 g de THF préalablement séché sur tamis moléculaire. Le milieu réactionnel ainsi obtenu est agité mécaniquement pendant 48 heures à une température de 60 °C. Après refroidissement à 20 °C, on filtre la résine et on la lave avec 500 ml d'eau, puis avec 250 ml d'eau à 60 °C. On la traite ensuite avec 300 ml d'une solution aqueuse de soude à 10 % et on lave à l'eau jusqu'à neutralité. La résine est lavée au méthanol (300 ml) puis séchée sous vide à 60 °C à poids constant.

On a effectué l'analyse élémentaire de la résine ainsi obtenue. Pour cette résine, N = 13,26 % en poids, soit 3,15 mmoles de fonction TBD/g de résine.

Résine indiquée par la suite: PS-DVB-TBD

**II Exemples de synthèse de mercapto-3-propionate de méthyle**

**II.1.Généralités**

Les essais ont été effectués dans un appareillage permettant d'étudier sous pression la réaction de formation du mercapto-3-propionate de méthyle à partir de l'acrylate de méthyle et de l'hydrogène sulfuré avec des résines basiques différentes comme catalyseurs.

L'introduction des réactifs avant le démarrage de la réaction permet d'effectuer la réaction selon un procédé batch (l'évolution de la réaction est équivalente aux conditions de fonctionnement en régime piston continu).

La conception de l'appareillage (description de l'appareillage: paragraphe II.2) permet d'étudier la réaction en régime batch agité (réacteur fermé) au moyen d'un réacteur tubulaire (résine en lit fixe) à travers lequel recircule à grand débit le milieu réactionnel liquide par l'intermédiaire d'une pompe sur le circuit d'une boucle de circulation qui est reliée aux deux extrémités du réacteur.

Cette technique opératoire de type batch agité permet d'étudier la cinétique de la réaction dans des conditions équivalentes à des conditions de fonctionnement en régime piston continu (réacteur ouvert), étant donné que la totalité des réactifs ($H_2S$ et acrylate de méthyle) est introduite dans l'appareillage, avant le démarrage de la réaction, le réacteur étant isolé (pas de contact avec la résine) (protocole opératoire : paragraphe II.3).

Le suivi de l'évolution de la réaction au cours du temps est effectué par prélèvement d'échantillons qui sont analysés par chromatographie en phase gazeuse pour déterminer la conversion de l'acrylate de méthyle et les sélectivités correspondantes en mercapto-3 propionate de méthyle et en thio-3,3' dipropionate de méthyle en fonction du temps.

**II.2 Appareillage.**

Comme représenté à la figure unique, l'appareillage en acier inoxydable se compose des éléments suivants :

- un réacteur tubulaire vertical 1, dans lequel est contenu la charge de résine fonctionnalisée 2,
- une boucle de recirculation 3 débouchant à l'extrémité supérieure 4 et à l'extrémité inférieure 5 du réacteur 1, cette boucle comprenant des conduits reliant successivement à partir de l'extrémité inférieure 5, une vanne de fermeture 6, une dérivation 7 équipée d'une vanne 8, un échangeur 9 à double enveloppe, une pompe à engrenage 10 (débit maximum 40 l/h), une prise de température 11, un débit mètre à bille 12, un réservoir cylindrique 13 à double enveloppe, muni d'un hublot 14 en verre épais transparent. Ce réservoir 13 est relié à l'extrémité supérieure 4 du réacteur par un conduit passant par une vanne de fermeture 15. Le réservoir 13 est placé au dessus du réacteur 1.

Cette boucle de circulation 3 comporte elle-même une boucle de dérivation 16 équipée de 2 vannes 17 et 18. Cette boucle 16 permet d'isoler le réacteur 1 du courant de circulation grâce à la coopération des vannes 6, 15, 17, 18.

Le réservoir 13 est équipé à sa partie supérieure d'un conduit 19 d'introduction de l'acrylate de méthyle. Ce conduit 19 comporte une vanne 20. Le réservoir 13 est également équipé d'un conduit 21 équipé d'un vanne de pression 22. Le conduit 21 est relié à une torche.

Le réservoir 13 est équipé à sa partie inférieure d'un conduit 23 équipé d'une vanne 24. et destiné à l'introduction de $H_2S$ sous pression dans le réservoir.

La partie inférieure du réservoir est reliée par un conduit 25 équipé d'une vanne 26 à un récepteur 27. Ce dernier est muni à sa partie inférieure d'un conduit 28 équipé d'une vanne 29. Le conduit 28 permet la récupération d'échantillons en cours de réaction.

**II.3.Protocole opératoire**

Les opérations de chargement de résine et d'introduction de l'acrylate de méthyle sont effectuées en atmosphère d'azote.

**II.3.1 Préparation des mélanges réactionnels**

Le réacteur 1, contenant la résine 2 (charges de l'ordre de 8 g), est isolé du reste de l'appareillage par la fermeture des vannes 6 et 15. L'acrylate de méthyle est introduit par le conduit 19 dans le réservoir cylindrique 13 qui est en communication directe avec la boucle de recirculation. L'appareillage est mis à une pression de 3 bars d'azote. Le réservoir cylindrique 13, dans lequel va être constitué le mélange réactionnel de départ est refroidi par une circulation d'huile à 5°C (provenant d'un cryothermostat) qui traverse aussi la double enveloppe externe de l'échangeur 9 de la boucle de recirculation. La pompe de circulation 10 est mise en route et le liquide contenu dans le réservoir cylindrique circule dans la boucle 3 et la boucle 16 en se dirigeant du réservoir 13 vers la vanne 17 avant de passer par la vanne 18.

L'hydrogène sulfuré, provenant d'une alimentation sous 16 bars de pression est injecté par le conduit 23 dans le réservoir 13 au moyen d'un diffuseur et se dissout dans l'acrylate de méthyle refroidi. A la fin de la l'injection de l'$H_2S$ la pression est de 13 bars et la température du mélange liquide (acrylate de méthyle + $H_2S$ ) est de 15 °C. Le volume de chargement peut être contrôlé par le hublot 14.

**II.3.2 Conduite des essais**

La consigne du cryothermostat est mise à la valeur correspondant à la température à laquelle la réaction doit être effectuée, et tandis que l'huile monte rapidement à la température fixée, la mélange réactionnel circulant est introduit par l'ouverture des vannes 15 et 6 et la fermeture des vannes 17 et 18 dans le réacteur 1 à travers lequel il recircule à débit élevé (maximum : 40 l/h). La température de réaction programmée pour l'essai est maintenue pendant la durée de la réaction. La pression de la phase gazeuse dans l'installation qui est reliée à la vanne de pression 22, s'établit entre 16 bars et 28 bars, suivant les conditions d'essai.

En cours d'essai, à des temps déterminés, des échantillons du milieu réactionnel sont prélevés par l'intermédiaire du récepteur 27 et récupérés à pression atmosphérique, puis analysés par chromatographie en phase gazeuse. A la fin de l'essai l'installation est décomprimée et le produit final de réaction est récupéré.

**II.4.Analyse des produits de réaction**

Les analyses chromatographiques en phase gazeuse (CG) sont effectuées avec un chromatographe Hewlett Packard 5890 FID équipé d'une colonne capillaire de 50 m contenant une phase réticulée diméthylpolysiloxane (origine HP Ultra-1).

**II.5 Essais expérimentaux**

**II.5.1 Conditions opératoires**

Les essais ont été effectués selon le protocole opératoire que nous avons mis en oeuvre et qui a été décrit dans un paragraphe précédent.
II.5.1.1 Résines

- Résine comparative:
  Résine Amberlyst® A-21 (Rohm et Haas) à fonction amine tertiaire(diméthylamino), donnée comme exemple dans le brevet US 5008432.
- Résine de l'invention:
  Résine à fonction 1,5,7-triazabicyclo[4.4.0.]déc-5-ène préparée selon le mode opératoire décrit ci-dessus et dénommée par la suite : PS-DVB-TBD.

II.5.1.2 Ratios molaires $H_2S$/Méthylacrylate:

4/1, ou 6/1, ou 8/1.

II.5.1.3 Températures:

15°C, ou 30°C, ou 45°C.

II.5.1.4 Pressions opératoires

Les pressions opératoires dépendent de l'excès de $H_2S$ et de la température de réaction.

Les pressions exercées sur le liquide du milieu réactionnel sont telles que le liquide est homogène ( pas de dégazage de $H_2S$ ).

Comme l'appareillage utilisé ne peut pas, pour des raisons de sécurité, fonctionner au dessus d'une pression de 29 bars, il n'a pas été possible d'effectuer d'essai à la température de 45°C avec un ratio molaire $H_2S$/méthylacrylate égal à 8/1, la pression opératoire pour cet essai devant dépasser 29 bars.

II.5.1.5 Suivi de la réaction

Des prélèvements du liquide du milieu réactionnel sont effectués à des temps déterminés et l'analyse chromatographique de ces échantillons permet de suivre l'avancement de la réaction et de déterminer la conversion de l'acrylate de méthyle (MA) en fonction du temps ainsi que les sélectivités correspondantes en mercapto-3 propionate de méthyle (MMP).

Compte tenu des faibles quantités de résines utilisées (charges de 8g) par rapport à la quantité de MA mise en jeu, et qui est identique pour chaque essai ( 172g, 2 moles), les vitesses de réaction sont relativement lentes, et au bout de 6 heures les conversions du MA ne sont pas complètes.

Pour les essais effectués à 30°C ou à 45°C, la réaction est poursuivie encore pendant 1/2 heure, puis l'appareillage est vidé après mise à la pression atmosphérique. Les bruts de réaction sont analysés par CPG. La durée totale de la réaction est de 6,5 heures.

Pour les essais effectués à 15°C, la réaction continu encore pendant une heure et demie avec un prélévement d'échantillon au bout de la $7^{ième}$ heure, puis l'appareillage est vidé et le brut de réaction est analysé par CPG.
La durée totale de la réaction est de 7,5 heures.

II.5.1.6 Présentation des résultats

Les résultats comparatifs obtenus avec les deux charges de résines ci-dessus ( Amberlyst® A-21 et PS-DVB-TBD) sont représentés dans les tableaux I et II suivants. Dans ces tableaux figurent le ratio molaire $H_2S$/MA, la température de réaction, la pression maximale de la réaction, les temps des prélèvements avec les valeurs correspondantes de la conversion de l'acrylate de méthyle (MA) et de la sélectivité en mercaptopropionate de méthyle (MMP). Le brut de réaction correspond au produit recueilli à la fin de la réaction.

II.5.1.6.1 Essais comparatifs avec la résine Amberlyst® A-21

| | |
|---|---|
| Charge de résine (sèche) | 8 g |
| Acrylate de méthyle | 172 g |
| Résultats | Tableau I |

On remarque que pour un même ratio molaire $H_2S$/MA, la sélectivité en MMP diminue lorsque la température de réaction augmente.

II.5.1.6.2 Essais selon la présente invention avec la résine

| PS-DVB-TBD | |
|---|---|
| Charge de résine (sèche) | 8 g |
| Acrylate de méthyle | 172 g |
| Résultats | Tableau II |

On remarque que la sélectivité en MMP est supérieure à celle obtenue précédemment et que cette sélectivité ne diminue pratiquement pas lorsque la température de réaction augmente.

TABLEAU I

| Essai | $H_2S$/MA | Temp. | Pression | 2 heures | | 4 heures | | 6 heures | | 7 heures | | Brut de réaction | |
|---|---|---|---|---|---|---|---|---|---|---|---|---|---|
| n° | | °C | Bars | Conv.MA (%) | Sél.MMP (%) | Conv.MA (%) | Sél.MMP (%) | Conv.MA (%) | Sél.MMP (%) | Conv.MA (%) | Sél.MMP (%) | Conv.MA (%) | Sél.MMP (%) |
| 1* | 4/1 | 15 | 16 | 58,6 | 93,4 | 83,6 | 91,6 | 94,5 | 91,4 | 97,9 | 91,0 | 98,5 | 90,9 |
| 2* | 4/1 | 30 | 20 | 78,8 | 89,9 | 93,4 | 89,6 | 97,6 | 89,6 | - | - | 98,9 | 89,5 |
| 3* | 4/1 | 45 | 28 | 85,7 | 88,7 | 95,5 | 88,6 | 98,3 | 88,5 | - | - | 98,3 | 88,5 |
| 4* | 6/1 | 15 | 17 | 51,9 | 95,4 | 71,1 | 95,2 | 88,8 | 95,1 | 95,5 | 95,0 | 98,4 | 94,9 |
| 5* | 6/1 | 30 | 22 | 74,4 | 94,0 | 91,7 | 93,6 | 96,9 | 93,4 | - | - | 98,8 | 93,3 |
| 6* | 6/1 | 45 | 28 | 79,5 | 92,8 | 92,9 | 92,5 | 97,2 | 92,4 | - | - | 98,5 | 92,3 |
| 7* | 8/1 | 15 | 20 | 41,0 | 94,9 | 62,9 | 94,8 | 80,9 | 94,6 | 89,0 | 94,6 | 95,8 | 94,6 |
| 8* | 8/1 | 30 | 24 | 59,5 | 94,7 | 82,7 | 94,5 | 93,8 | 94,4 | - | - | 98,1 | 94,1 |

* Essais comparatifs

TABLEAU II

| Essai | $H_2S/MA$ | Temp. | Pression | 2 heures | | 4 heures | | 6 heures | | 7 heures | | Brut de réaction | |
|---|---|---|---|---|---|---|---|---|---|---|---|---|---|
| n° | | °C | Bars | Conv.MA (%) | Sél.MMP (%) | Conv.MA (%) | Sél.MMP (%) | Conv.MA (%) | Sél.MMP (%) | Conv.MA (%) | Sél.MMP (%) | Conv.MA (%) | Sél.MMP (%) |
| 9 | 4/1 | 15 | 16 | 64,6 | 94,2 | 85,7 | 94,1 | 95,4 | 94,0 | 98,8 | 93,9 | 99,4 | 93,6 |
| 10 | 4/1 | 30 | 20 | 89,8 | 93,8 | 96,4 | 93,6 | 98,5 | 93,5 | - | - | 99,3 | 93,5 |
| 11 | 4/1 | 45 | 28 | 92,9 | 93,6 | 97,1 | 93,3 | 98,9 | 93,2 | - | - | 99,5 | 93,1 |
| 12 | 6/1 | 15 | 17 | 54,2 | 96,1 | 68,9 | 96,0 | 87,5 | 95,9 | 94,9 | 95,8 | 98,1 | 95,8 |
| 13 | 6/1 | 30 | 22 | 82,0 | 95,9 | 92,8 | 95,5 | 97,1 | 95,5 | - | - | 99,0 | 95,5 |
| 14 | 6/1 | 45 | 28 | 83,3 | 95,4 | 93,8 | 95,2 | 98,1 | 95,1 | - | - | 98,9 | 95,0 |
| 15 | 8/1 | 15 | 20 | 40,7 | 96,9 | 61,7 | 96,8 | 80,3 | 96,8 | 89,1 | 96,8 | 97,8 | 96,8 |
| 16 | 8/1 | 30 | 24 | 74,7 | 97,1 | 90,0 | 96,8 | 96,1 | 96,7 | - | - | 98,6 | 96,7 |

## Revendications

**1.** Procédé de synthèse d'ester de l'acide mercapto-3 propionique par réaction d'addition de $H_2S$ à l'ester correspondant de l'acide acrylique en présence d'un support solide possédant des groupes fonctionnels basiques, caractérisé en ce que les groupes fonctionnels sont des groupes guanidine, à la condition que ceux-ci soient dépourvus d'hydrogène directement lié à un atome d'azote.

**2.** Procédé suivant la revendication 1, caractérisé en ce que ces groupes fonctionnels sont choisis parmi :

1° un radical guanidine de formule générale (C) :

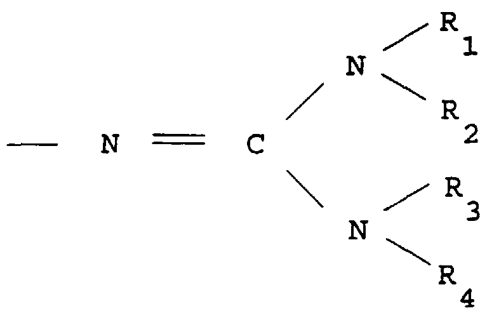

(C)

dans laquelle $R_1$, $R_2$, $R_3$, $R_4$ sont indépendamment l'un de l'autre des groupes hydrocarbonés tels que méthyle, éthyle, propyle, butyle, l'azote imine étant lié au support solide par une liaison chimique ou une succession de liaisons chimiques,
2° un radical guanidine bicyclique de formule (D) :

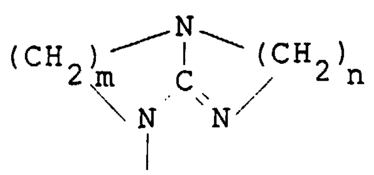

(D)

dans laquelle m et n valent chacun de 2 à 4, avec la condition que n soit inférieur ou égal à m, ce radical (D) étant lié au support solide par une liaison chimique ou une succession de liaisons chimiques partant de l'azote initialement N-H de la guanidine bicyclique correspondante.

**3.** Procédé suivant la revendication 2, caractérisé en ce que le radical (D) est choisi parmi les radicaux dérivés des guanidines suivantes: 1,5,7-triazabicyclo [4.3.0] non-6-ène (m = 3, n = 2), 1,5,7-triazabicyclo [4.4.0] déc-5-ène (m = 3, n = 3), 1,6,8-triazabicyclo [5.3.0] déc-7-ène (m = 4, n = 2), 1,4,6,-triazabicyclo [3.3.0] oct-4-ène (m = 2, n = 2).

**4.** Procédé suivant la revendication 2 ou 3, caractérisé en ce que la résine est à base de polystyrène divinylbenzène (PS-DVB) ayant la formule générale (I) :

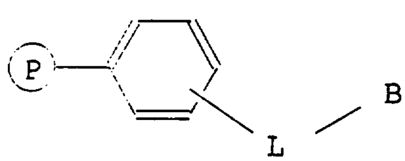

(I)

B étant un groupe choisi parmi les radicaux de formule générale (C), (D), L étant un radical organique linéaire ayant une longueur égale ou supérieure à celle du radical méthylène $-(CH_2-)$ et notamment le radical méthylène,

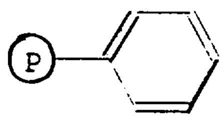

étant le support résine PS-DVB.

**5.** Procédé suivant la revendication 4, caractérisé en ce que :

- le radical (C) est substitué par L, ce dernier représentant alors un radical $-CH_2-$, et $R_1$, $R_2$, $R_3$ et $R_4$ représentant chacun un groupe méthyle,
- le radical (D) est substitué par L sur l'azote qui dans le composé bicyclique apparenté porte un hydrogène, avec la condition que L représente alors un radical $-(CH_2)_p-$, p entier valant 1 à 9,

**6.** Procédé suivant la revendication 4, caractérisé en ce que la résine polymérique a la formule générale (II):

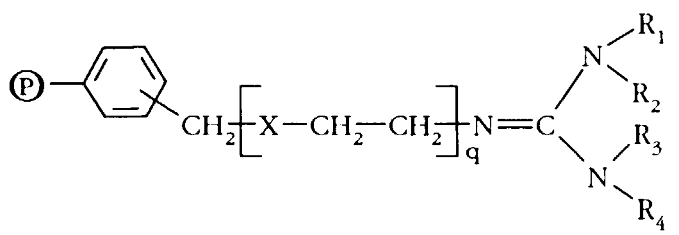

(II)

dans laquelle X représente l'atome d'oxygène ou de soufre, q vaut 1 ou 2,
$R_1$, $R_2$, $R_3$ et $R_4$ sont indépendamment l'un de l'autre choisis parmi les groupes méthyle, éthyle, propyle, butyle.

**7.** Procédé suivant la revendication 6, caractérisé en ce que $R_1$, $R_2$, $R_3$ et $R_4$ représentent chacun un groupe méthyle et que q vaut 1.

**8.** Procédé suivant l'une des revendication 2 à 5, caractérisé en ce que la résine fonctionnalisée a la formule générale (III):

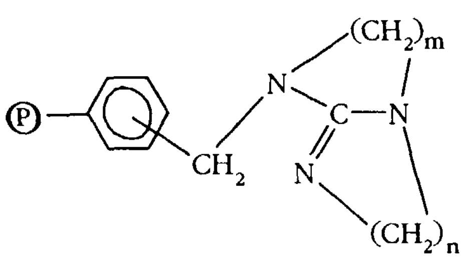

(III)

dans laquelle n vaut 2 ou 3 et m vaut 2, 3 ou 4, avec la condition que n soit inférieur ou égal à m.

**9.** Procédé suivant l'une des revendication 2 à 7, caractérisé en ce que le rapport molaire $H_2S$/ester de l'acide acrylique est de 3 à 10.

**10.** Procédé suivant l'une des revendication 1 à 9, caractérisé en ce que la pression réactionnelle est de 15 bars (1500 kPa) à 35 bars (3500 kPa).

**11.** Procédé suivant l'une des revendication 1 a 10, caractérisé en ce que la réaction est effectuée à une température de 15°C à 80°C.

**12.** Procédé suivant la revendication 11, caractérisé en ce que la réaction est effectuée à une température de 15°C à 45°C.

**Patentansprüche**

**1.** Verfahren zur Herstellung eines 3-Mercaptopropionsäureesters durch Anlagerung von $H_2S$ an den entsprechenden Acrylsäureester in Gegenwart eines festen Trägers mit funktionellen basischen Gruppen, dadurch gekennzeichnet, daß die funktionellen Gruppen Guanidingruppen sind, unter der Bedingung, daß diese keine unmittelbar an ein Stickstoffatom gebundene Wasserstoffatome haben.

**2.** Verfahren nach Anspruch 1, dadurch gekennzeichnet, daß diese funktionellen Gruppen ausgewählt sind aus:

1° einem Guanidinrest der allgemeinen Formel (C):

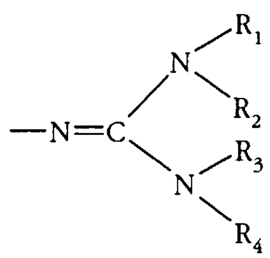

(C)

in der $R_1$, $R_2$, $R_3$, $R_4$ unabhängig voneinander Kohlenwasserstoffgruppen sind wie Methyl-, Ethyl-, Propyl-, Butylgruppen und das Stickstoffimin durch eine chemische Bindung oder eine Folge von chemischen Bindungen an den festen Träger gebunden ist,
2° einem bicyclischen Guanidinrest der Formel (D):

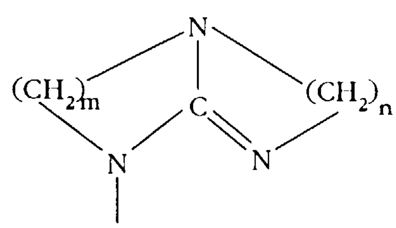

(D)

in der m und n jeweils eine Zahl von 2 bis 4 sind, unter der Bedingung, daß n kleiner oder gleich m ist und dieser Rest (D) ausgehend vom ursprünglichen N-H-Stickstoff des entsprechenden bicyclischen Guanidins durch eine chemische Bindung oder eine Folge von chemischen Bindungen an den festen Träger gebunden ist.

**3.** Verfahren nach Anspruch 2, dadurch gekennzeichnet, daß der Rest (D) aus den folgenden Guanidinderivatresten gewählt ist:

1,5,7-Triazabicyclo[4.3.0]non-6-en (m = 3, n = 2),
1,5,7-Triazabicyclo[4.4.0]dec-5-en (m = 3, n = 3),
1,6,8-Triazabicyclo[5.3.0]dec-7-en (m = 4, n = 2),
1,4,6-Triazabicyclo[3.3.0]oct-4-en (m = 2, n = 2).

**4.** Verfahren nach Anspruch 2 oder 3, dadurch gekennzeichnet, daß das Harz auf der Grundlage von Polystyrol-Divinylbenzol (PS-DVB) die allgemeine Formel (I) hat:

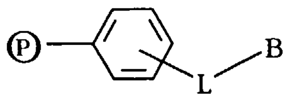

(I)

in der B eine Gruppe gewählt aus den Resten der allgemeinen Formeln (C) und (D) ist, L ein organischer linearer Rest einer Länge gleich oder größer der des Methylenrestes -($CH_2$-), und insbesondere der Methylenrest ist und

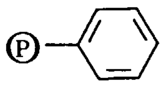

das Trägerharz PS-DVB ist.

**5.** Verfahren nach Anspruch 4, dadurch gekennzeichnet, daß:

- der Rest (C) mit L substituiert ist, wobei letzterer dann einen -$CH_2$-Rest bedeutet und $R_1$, $R_2$, $R_3$ und $R_4$ jeweils eine Methylgruppe bedeuten,
- der Rest (D) mit L an dem Stickstoff substituiert ist, der in der entsprechenden bicyclischen Verbindung ein Wasserstoffatom besitzt, unter der Bedingung, daß L dann einen -$(CH_2)_p$-Rest bedeutet, in dem p eine ganze Zahl von 1 bis 9 ist.

**6.** Verfahren nach Anspruch 4, dadurch gekennzeichnet, daß das Polymerharz die allgemeine Formel (II) hat:

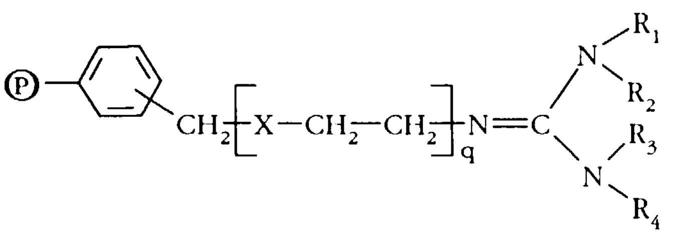

(II)

in der X ein Sauerstoff- oder Schwefelatom bedeutet und q gleich 1 oder 2 ist und $R_1$, $R_2$, $R_3$ und $R_4$ unabhängig voneinander aus Methyl-, Ethyl-, Propyl- oder Butylgruppen gewählt sind.

**7.** Verfahren nach Anspruch 6, dadurch gekennzeichnet, daß $R_1$, $R_2$, $R_3$ und $R_4$ jeweils eine Methylgruppe bedeuten und q gleich 1 ist.

**8.** Verfahren nach einem der Ansprüche 2 bis 5, dadurch gekennzeichnet, daß das funktionell wirksam gemachte Harz die allgemeine Formel (III) hat:

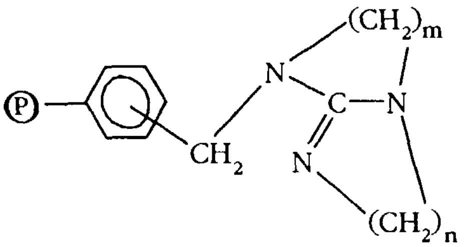

(III)

in der n gleich 2 oder 3 und m gleich 2, 3 oder 4 ist, vorausgesetzt, daß n kleiner oder gleich m ist.

**9.** Verfahren nach einem der Ansprüche 2 bis 7, dadurch gekennzeichnet, daß das Molverhältnis $H_2S$/Acrylsäureester 3 bis 10 ist.

**10.** Verfahren nach einem der Ansprüche 1 bis 9, dadurch gekennzeichnet, daß der Reaktionsdruck 15 bar (1.500 kPa) bis 35 bar (3.500 kPa) ist.

**11.** Verfahren nach einem der Ansprüche 1 bis 10, dadurch gekennzeichnet, daß die Reaktion bei einer Temperatur von 15 °C bis 80 °C durchgeführt wird.

**12.** Verfahren nach Anspruch 11, dadurch gekennzeichnet, daß die Reaktion bei einer Temperatur von 15 °C bis 45 °C durchgeführt wird.

**Claims**

**1.** Process for the synthesis of 3-mercaptopropionic acid esters by addition reaction of $H_2S$ to the corresponding acrylic acid ester in the presence of a solid support possessing basic functional groups, characterized in that the functional groups are guanidine groups, on condition that these groups are free of hydrogen that is directly attached to a nitrogen atom.

**2.** Process according to Claim 1,
characterized in that these functional groups are chosen from:

1. a guanidine radical of general formula (C) :

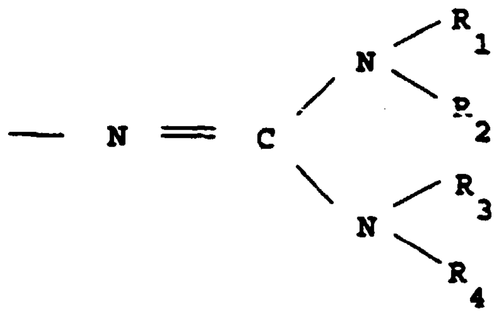

(C)

in which $R_1$, $R_2$, $R_3$ and $R_4$ are, independently of each other, hydrocarbon groups such as methyl, ethyl, propyl or butyl, the imine nitrogen being attached to the solid support by a chemical bond or a series of chemical bonds,
2. a bicyclic guanidine radical of formula (D):

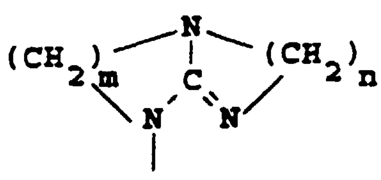

(D)

in which m and n are each from 2 to 4, with the condition that n is less than or equal to m, this radical (D) being attached to the solid support by a chemical bond or a series of chemical bonds starting from the initially N-H nitrogen of the corresponding bicyclic guanidine.

**3.** Process according to Claim 2,
characterized in that the radical (D) is chosen from the following guanidine radicals: 1,5,7-triazabicyclo[4.3.0]non-6-ene (m = 3, n = z), 1,5,7-triazabicyclo[4.4.0]dec-5-ene (m = 3, n = 3), 1,6,8-triazabicyclo[5.3.0]dec-7-ene (m = 4, n = 2), 1,4,6-triazabicyclo[3.3.0]oct-4-ene (m = 2, n = 2).

**4.** Process according to Claim 2 or 3,
characterized in that the resin is based on polystyrene-divinylbenzene (PS-DVBj having the general formula (I):

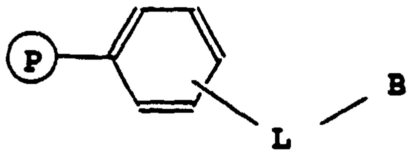

(I)

B being a group chosen from radicals of general formula (C) and (D), L being a linear organic radical which is as long as or longer than the methylene radical $-(CH_2)-$ and in particular the methylene radical,

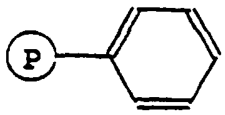

being the PS-DVB resin support.

**5.** Process according to Claim 4,
characterized in that:

- the radical (C) is substituted with L, the latter then representing a $-CH_2-$ radical, and $R_1$, $R_2$, $R_3$ and $R_4$ each representing a methyl group,
- the radical (D) is substituted with L on the nitrogen which, in the related bicyclic compound, bears a hydrogen, with the condition that L then represents a radical $-(CH_2)_p-$, p being an integer equal to 1 to 9.

**6.** Process according to Claim 4,
characterized in that the polymeric resin has the general formula (II) :

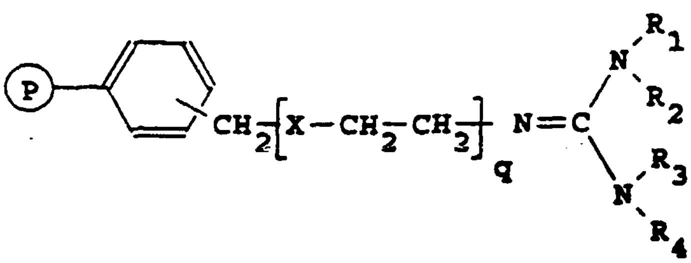

(II)

in which X represents an oxygen or sulphur atom, q is 1 or 2 and $R_1$, $R_2$, $R_3$ and $R_4$ are, independently of each other, chosen from methyl, ethyl, propyl and butyl groups.

**7.** Process according to Claim 6,
characterized in that $R_1$, $R_2$, $R_3$ and $R_4$ each represent a methyl group and q is 1.

**8.** Process according to one of Claims 2 to 5, characterized in that the functionalized resin has the general formula (III):

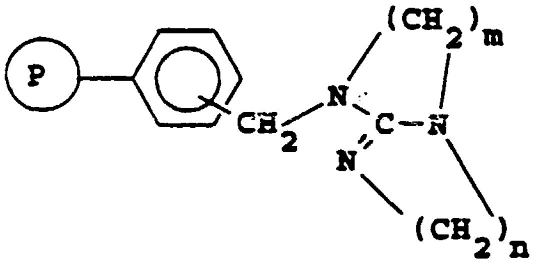

(III)

in which n is 2 or 3 and m is 2, 3 or 4, with the condition that n is less than or equal to m.

**9.** Process according to one of Claims 2 to 7, characterized in that the $H_2S$/acrylic acid ester molar ratio is from 3 to 10.

**10.** Process according to one of Claims 1 to 9, characterized in that the reaction pressure is from 15 bar (1500 kPa) to 35 bar (3500 kPa).

**11.** Process according to one of Claims 1 to 10, characterized in that the reaction is carried out at a temperature of from 15°C to 80°C.

**12.** Process according to Claim 11, characterized in that the reaction is carried out at a temperature of from 15°C to 45°C.

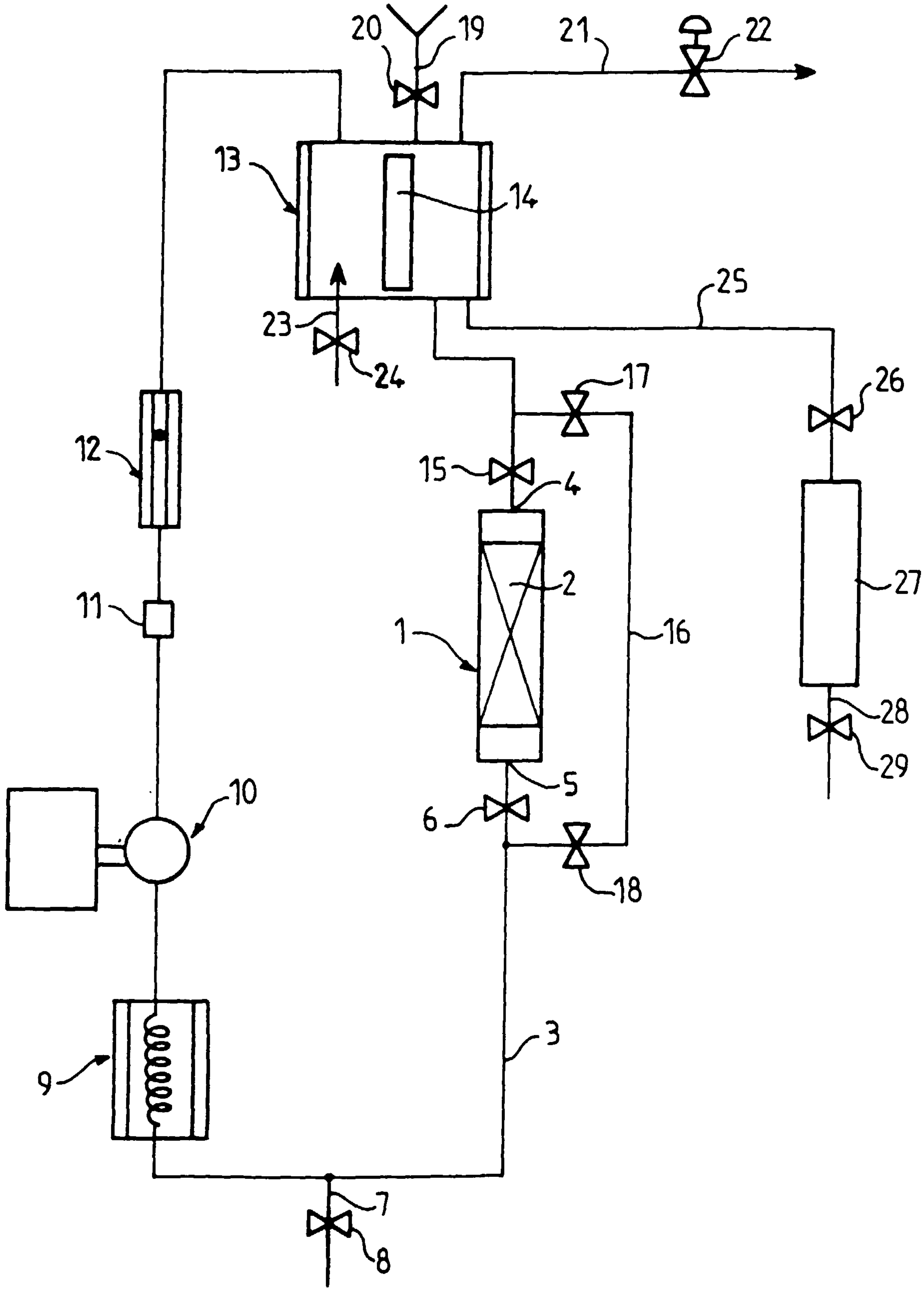
20
19
21
22
13
14
25
23
24
17
26
12
15
4
2
27
11
1
16
10
5
28
29
6
18
3
9
7
8